(19) Europäisches Patentamt
European Patent Office
Office européen des brevets

(11) **EP 3 822 626 A1**

(12) **EUROPEAN PATENT APPLICATION**
published in accordance with Art. 153(4) EPC

(43) Date of publication:
**19.05.2021 Bulletin 2021/20**

(21) Application number: **19833762.8**

(22) Date of filing: **10.07.2019**

(51) Int Cl.:
*G01N 27/327* (2006.01)       *G01N 27/447* (2006.01)
*G01N 33/543* (2006.01)

(86) International application number:
**PCT/KR2019/008461**

(87) International publication number:
**WO 2020/013590 (16.01.2020 Gazette 2020/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **11.07.2018 KR 20180080348**

(71) Applicant: **XYZ Platform Inc.**
**Seoul 04783 (KR)**

(72) Inventors:
• **HWANG, Kyo Seon**
  **Seoul 02799 (KR)**
• **KIM, Hye Jin**
  **Seoul 06081 (KR)**

(74) Representative: **Hwang, Jae Suk et al**
**Venner Shipley LLP**
**200 Aldersgate**
**London EC1A 4HD (GB)**

(54) **MICROELECTRODE BIOSENSOR USING DIELECTROPHORESIS AND METHOD FOR DETECTING BIOLOGICAL MATERIAL BY USING SAME**

(57)     A biosensor of the present invention comprises: a first microelectrode and a second microelectrode arranged to intersect in a comb shape on a substrate; and a plurality of receptors fixed in a space between the microelectrodes to specifically react with a target biomaterial. In particular, a micropattern of a conductive material is formed in the space between the microelectrodes. Accordingly, greater electric field intensity can be obtained compared to a biosensor without micropatterns, thereby concentration of the target biomaterial using dielectric electrophoretic forces can be performed more efficiently. In addition, damage to biomolecules can be prevented by lowering the intensity of a voltage for a dielectric electrophoresis phenomenon and the biosensor can be easily commercialized as a health care sensor for diagnosing diseases.

FIG. 2

(a)

(b)

**Description**

## TECHNICAL FIELD

The present invention relates to a biosensor and more particularly to a dielectrophoresis-used microelectrode biosensor configured to further improve sensitivity and detection width of a sensor by forming, between microelectrodes, a receptor specifically reacting with a target biomaterial and by increasing a probability of specifically reacting with a target biomaterial using concentration effect based on dielectrophoresis phenomenon, and a method for detecting a bio material using the same.

## BACKGROUND OF THE INVENTION

Recently, bio sensors are developed on a large scale configured to detect, by an electric method, presence and absence of various biomaterials such genes and proteins, and concentrations thereof.

One of these examples is to use an IDE (Interdigitated Electrode), and the IDE is so evaluated as to properly measure concentration of biomaterials even if a region where receptors fixed to specifically react with biomaterials is substantially very broad in a zigzag shape.

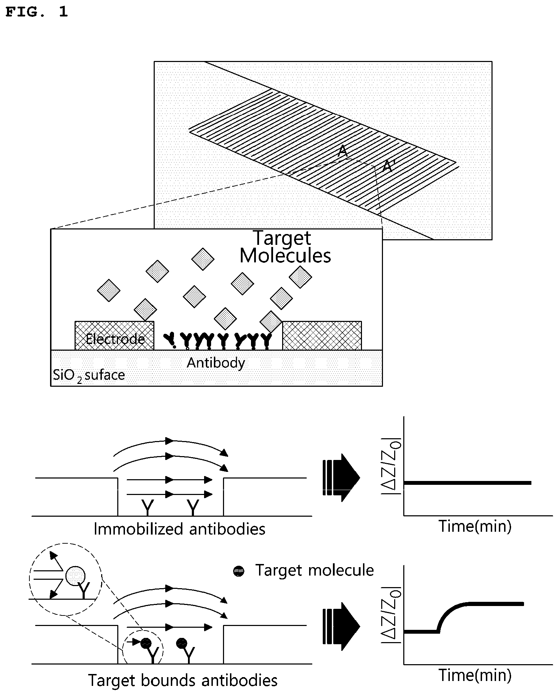

Referring to FIG. 1, a basic IDE sensor is arranged with metal electrodes in a comb shape on both sides, to detect a target by measuring a change in impedance generated between adjacent electrodes.

That is, an impedance component is determined by allowing an antibody to be fixed between electrodes to form an electric field, where disturbance is generated on an electric field formed between electrodes by allowing target molecules to be positioned by pushing out a bio buffer solution when the target molecules are specifically fixed to the antibody, which resultantly induces the change in impedance.

An amount of change in impedance may be determined by an amount of target biomolecules, whereby quantitative analysis of target biomolecules within a sample is made to be enabled by ascertaining the amount of change in impedance.

Meantime, in order to improve the sensitivity of the said IDE sensor, a technology is disclosed by a registered patent No.: 10-1727107 entitled as 'microelectrode bio-sensor using dielectrophoresis' to allow an IDE sensor to use a dielectrophoresis phenomenon.

Here, the 'dielectrophoresis (dielectric electrophoresis)' is a phenomenon in which a net force is exerted in an electric field by allowing a bipolarity to be induced on particles when non-polar particles exist in non-uniform AC electrical field.

The strength of dielectrophoresis force may be increased by adjusting a distance between electrodes of the IDE sensor or a size of non-uniform electrical field formed through the intensity change of applied voltage.

However, in a case when a biosensor is so formed as to use the dielectrophoresis, there is generated a possibility of incurring a damage to a bio particle when the intensity of voltage applied in order to obtain the dielectrophoresis is increased.

Furthermore, in consideration of applications in various aspects including commercialization of IDE sensor as a health care sensor for disease diagnosis, or manufacturing of the same for portability, the increase in the intensity of voltage in order to obtain a dielectrophoresis effect may create another problem.

[Prior art document]

[Patent document]

Korea registered patent No.:1727107 (Published on April 17, 2017)

## DETAILED DESCRIPTION OF THE INVENTION

## TECHNICAL SUBJECT

The present invention is provided to solve the aforementioned problems and it is an object of the present invention to provide a dielectrophoresis-used microelectrode bio sensor configured to further improve sensitivity and detection width of a sensor by increasing a probability of specifically reacting with a target bio material through concentration effect using the dielectrophoresis to thereby lower the intensity of voltage for use of the dielectrophoresis.

It is another object of the present invention to provide a method for detecting a bio material through a bio sensor using the dielectrophoresis.

## TECHNICAL SOLUTION

In one general aspect of the present invention, there may be provided a dielectrophoresis-used microelectrode bio sensor comprising:

a first microelectrode with a plurality of first protruding electrodes arranged in a comb shape on a substrate;

a second microelectrode arranged to intersect with each first protruding electrode formed on the first microelectrode and arranged with a plurality of second protruding electrodes arranged in a comb shape;

a conductive-materialed micropattern formed in a space between the first microelectrode and the second microelectrode; and

a plurality of receptors fixed in a space between the first microelectrode and the second microelectrode to specifically react with a target biomaterial.

Preferably, but not necessarily, the first microelectrode and the second microelectrode may be applied with an AC (Alternating Current) for generating dielectrophoresis forces.

Preferably, but not necessarily, the micropattern may be formed in any one shape of square, rectanglular and line(ar) shapes. Preferably, but not necessarily, size of AC for generating the dielectrophoresis may be greater than 0.25V but less than 0.35V, and frequency may be 50MHz.

In another general aspect of the present invention, there may be provided method for detecting biomaterial using a biosensor, comprising:

applying a voltage to the first microelectrode and the second microelectrode using the dielectrophoresis according to any one preceding claim of 1 to 8;

measuring an impedance between the first microelectrode and the second microelectrode; and

calculating one or more of presence or absence of target biomaterial and concentration thereof based on the measured impedance.

## ADVANTAGEOUS EFFECTS

According to the present invention, a probability capable of specifically reacting with a target biomaterial can be increased through concentration effect using the dielectric electrophoresis, whereby sensitivity and detection width of a biosensor can be enhanced.

Because target biomaterials inside a sample are collected, the impedance signal of the biosensor can be increased, which leads to increase in sensitivity of the biosensor whereby non-specific formation can be effectively inhibited to thereby exhibit a greater effect in an environment like plasma or serum.

Particularly, a greater electric field intensity can be obtained compared with a biosensor without micropattern because of use of micropattern between electrodes, whereby target biomaterials can be more effectively concentrated. Furthermore, damage to biomolecules can be prevented by lowering the intensity of voltage for dielectric electrophoresis, and the biosensor can be easily applied to various ways including commercialization or portability of the biosensor as a health care sensor for disease diagnosis.

## BRIEF DESCRIPTION OF THE DRAWINGS

FIG. 1 is an example explaining a basic principle of an IDE sensor,

FIG. 2 is a biosensor according to an exemplary embodiment of the present invention,

FIG. 3 is a micropattern according to an exemplary embodiment of the present invention,

FIGS. 4 and 5 are examples explaining a particle movement state according to positive and negative electrophoresis,

FIG. 6 is an example explaining a target molecule concentration phenomenon using dielectrophoresis forces and an impedance signal change of an IDE sensor,

FIG. 7 is an example explaining dielectrophoresis phenomenon in response to presence or absence of micropattern,

FIG. 8 is a method for detecting a biomaterial using a biosensor according to an exemplary embodiment of the present invention,

FIG. 9 is an example of a simulation result establishing a biomolecule concentration condition,

FIG. 10 is an example explaining an intensity of non-uniform AC electric field in response to voltage applied to microelectrodes,

FIG. 11 is an example explaining a biosensor used in the experiments,

FIG. 12 is an example on manufacturing process of biosensor,

FIG. 13 is a real physical example of a biosensor according to the present invention,

FIG. 14 is an example of a photograph after surface treatment ascertained by using a surface treatment method and a fluorescence detection method of sensor,

FIG. 15 is an example showing a dielectrophoresis voltage condition in response to the size of biomolecule,

FIG. 16 is an example explaining an amount of impedance change by dielectrophoresis effect in response to an applied voltage,

FIG. 17 is an example of an ascertained result of dielectrophoresis in relation to amyloid beta quantitative analysis,

FIG. 18 is an example of sensitivity analysis of an IDE sensor in response to dielectric electrophoresis effect, and

FIG. 19 is an example of amyloid beta quantitative analysis within plasma.

## BEST MODE

Exemplary embodiments of the present invention will be described in detail with reference to the accompanying drawing so as to allow a person skilled in the art to which the present invention belongs to easily implement the present invention. However, it should be understood that the present invention is not limited to particular embodiments, but encompasses all changes, modifications, equivalents and substitutes included within the ideas and technical scopes of the present invention. In describing the present invention, detailed descriptions of well-known technologies are omitted for brevity and clarity so as not to obscure the description of the present invention with unnecessary detail.

It will be further understood that the terms "comprises" and/or "comprising," or "includes" and/or "including" when used in this specification, specify the presence of stated features, regions, integers, steps, operations, elements, and/or components, but do not preclude the presence or addition of one or more other features, regions, integers, steps, operations, elements, components, and/or groups thereof.

As may be used herein across the specification, the terms "about", "approximately" and "substantially" are used to provide a meaning approximate to a number or a number adjacent to that number, when an intrinsic manufacturing error and material allowable error are provided, and, in order to assist understanding of the present invention, may be used to prevent an unscrupulous infringer from using the disclosed contents mentioned with accurate or absolute numbers unlawfully

It will be understood that, although the terms first, second, etc. may be used herein to describe various elements, these elements should not be limited by these terms. These terms are only used to distinguish one element from another.

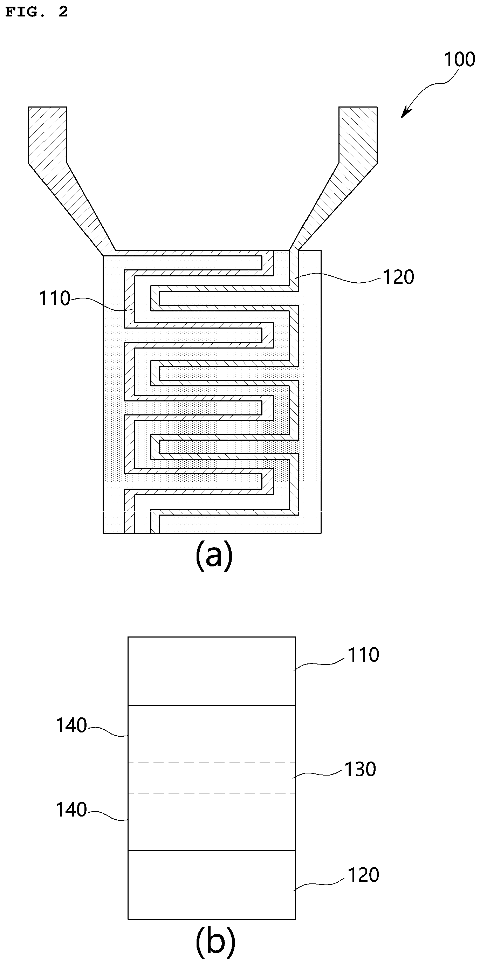

FIG. 2 is a biosensor (100) according to an exemplary embodiment of the present invention, where FIG. 2b shows an enlargement of dotted portion of FIG. 2a.

The biosensor (100) may include a first microelectrode (110), a second microelectrode (120), a micropattern (130), and a plurality of receptors.

As illustrated in FIG. 2a, the first microelectrode (110) may be arranged on a substrate in a comb shape with a plurality of first protruding electrodes, and the second microelectrode (120) may be arranged on a substrate in a comb shape with a plurality of second protruding electrodes.

At this time, each first protruding electrode formed on the first microelectrode (110) and each second protruding electrode formed on the second microelectrode (120) may be structurally arranged to mutually intersect.

A reaction region (140) may be formed in a space between the first microelectrode (110) and the second microelectrode (120) .

As shown in FIG. 2b, the micropattern (130) may be formed at a space formed between the first microelectrode (110) and the second microelectrode (120), and the micropattern may be formed with a conductive material, and may be formed in various patterns and shapes.

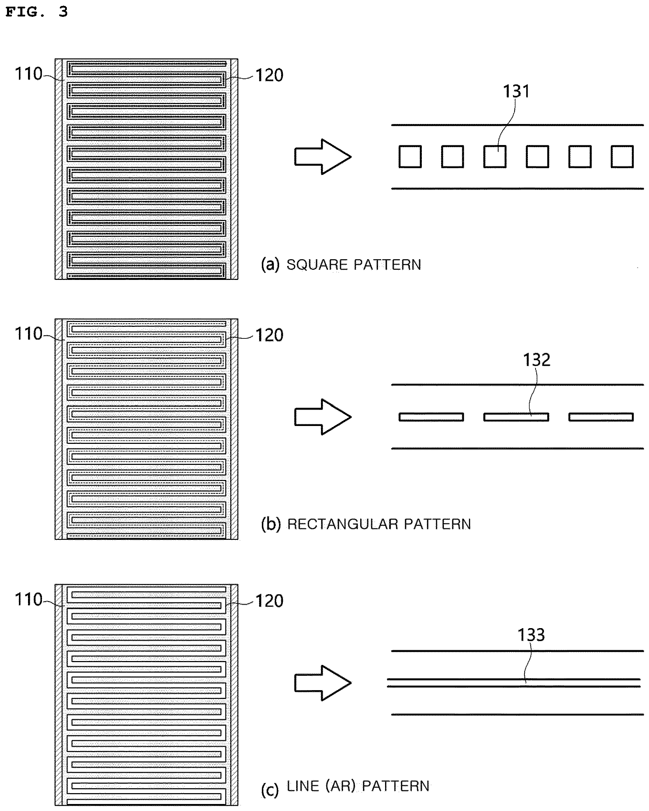

The micropattern (130) may be formed in any one shape of a square, a rectangle and a line(ar) shape as shown in FIG. 3. However, the present invention is not limited thereto.

FIG. 3a is an example of a square-shaped micropattern (131) intermittently formed at a middle portion in a space between the first microelectrode (110) and the second microelectrode (120) along the electrodes, and FIG. 3b is an example of a rectangle-shaped micropattern (132) intermittently formed at a middle portion in a space between the first microelectrode (110) and the second microelectrode (120) along the electrodes.

Furthermore, FIG. 3c is an example of a line(ar)-shaped micropattern (133) continuously formed at a middle portion in a space between the first microelectrode (110) and the second microelectrode (120) along the electrodes.

The square-shaped micropattern (131), the rectangular-shaped micropattern (132), and the line(ar)-shaped micropattern (133) may come in various sizes and shapes.

As a more detailed example, the square-shaped micropattern (131) may be formed with a length of about $2.5\mu m$ for each side. However, the present invention is not limited thereto. For example, each side of the square-shaped micropattern (131) may be formed with a length of greater than $2.0\mu m$ but less than $3.0\mu m$.

Furthermore, the rectangular-shaped micropattern (132) may be formed with a length of about $2.5\mu m$ for one side and of about 7.5pm for other sides. However, the present invention is not limited thereto. For example, one side of the rectangular-shaped micropattern (132) may be formed with a length of greater than $2.0\mu m$ but less than $3.0\mu m$ while the other sides may be formed with a length of greater than $7.0\mu m$ but less than $8.0\mu m$.

The width of the line(ar)-shaped micropattern (1331) may be formed with about $2.5\mu m$. However, the present invention is not limited thereto. For example, the line(ar)-shaped micropattern (133) may be formed with a width greater than $2.0\mu m$ but less than $3.0\mu m$.

The plurality of receptors may be fixed in a space between the first microelectrode (110) and the second microelectrode (120) to function as specifically reacting to a target biomaterial.

The receptor and the target biomaterial to which the receptor specifically reacts may be formed in a variety way. As one of the examples, the target biomaterial detected by using a biosensor (100) may be amyloid beta antibody, where the receptor may be amyloid beta antibody, aptamer, peptide and the like.

In light of impedance detection characteristic of biosensor using reaction of a target biomaterial, an impedance between the first microelectrode (110) and the second microelectrode (120) may be expressed in the following Equation 1.

[Equation 1]

$$Z = R + jX = R + j(XL-XC) = R - jXC = R - j(1/wC)$$

Where, Z is an impedance, R is a resistance, X is a reactance, C is a capacitance, and w is an angular frequency. The reactance X may be divided to an inductor component XL and a capacitor component XC, where because the first microelectrode (110) and the second microelectrode (120) are directly connected electricity-wise, it can be said that the inductor component (XL) is disregarded, and only the capacitor component (XC) exists.

When a plurality of 5 (five) receptors is fixedly arranged in a space between the first microelectrode (110) and the second microelectrode (120), most of the electric fields and impedance changes are generated to a horizontal direction where the first microelectrode (110) and the second microelectrode (120) are arranged across the plurality of receptors. An amount of target biomaterials can be detected by ascertaining the changes in the said resistance and reactance.

That is, when the plurality of receptors is fixed in a space between the first microelectrode (110) and the second microelectrode (120), and when the changes in the impedance at a space faced by the first microelectrode (110) and the second microelectrode (120) are ascertained when the target biomaterial reacts to the receptor, it is possible to qualitatively analyze the target biomaterial.

Meantime, an AC may be applied to the first microelectrode (110) and the second microelectrode (120) in order to generate the dielectric electrophoretic forces.

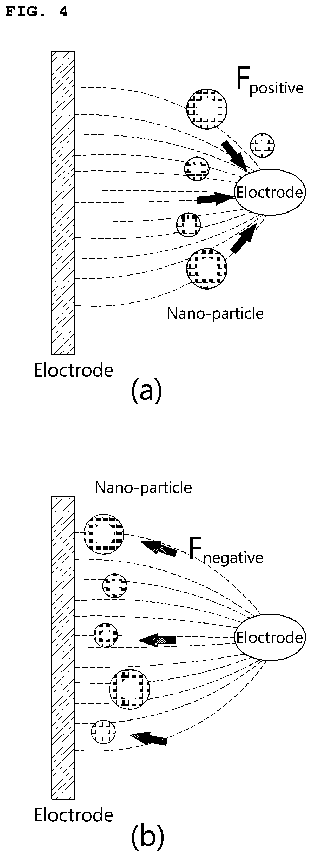

Referring to FIG. 4, a force to a predetermined direction exists in a non-uniform electric field unlike a case where an electrical field gradient is uniform. The di-electrophoresis may be defined as a phenomenon in which a net force is exerted in an electrical field by allowing a bipolarity to be induced on particles when non-polar particles exist in non-uniform AC electric field.

Here, the induced net force may be defined as DEFs (Di-electrophoresis Forces).

That is, when an AC voltage is applied to the biosensor (100), a non-uniform AC electric field is electrically formed, and non-polar particles existing within the electric field may possess the bipolarity to be affected by the DEFs to a predetermined direction.

The intensity (size) and direction of DEFs induced to each particle forming the target biomaterials may differ depending on dielectric properties including, but not limited to, voltage of electric field, frequency, conductivity ($\sigma$) of particle and medium and permittivity ($\epsilon$).

Therefore, a force that a spherical particle receives by the di-electrophoresis forces may be expressed by the following Equation 2.

[Equation 2]

$$F_{DEP} = 2\pi\varepsilon_m r^3 \, Re[K(\omega)] \, \nabla |Ersm|^2$$

Where, $\epsilon$m is permittivity of medium, r is a radius of a particle, Re[k($\omega$)] is a real number portion of Clausius Mossotti factor, and Ersm means a root-mean square of electric field.

At this time k($\omega$) may be determined by the following Equation 3 based on a relative permittivity ($\epsilon^*$ p) of particle and relative permittivity ($\epsilon * $m) of medium, and polarity of particle may be determined by the value thereof.

[Equation 3]

$$K(\omega) = \frac{\varepsilon_p^* - \varepsilon_m^*}{\varepsilon_p^* + 2\varepsilon_m^*}$$

When k($\omega$) value is greater than 0, the particle may move to a direction where the electrical field gradient is great by receiving the force. Conversely, when k($\omega$) value is smaller than 0, the particle may move to a direction where the electric field gradient is small in response to a formed shape of electrical field. The said phenomenon may be respectively called positive and negative DEP (Di-electrophoresis).

Thus, when a voltage is applied to allow the di-electrophoresis forces to be generated by the electric field between the first microelectrode (110) and the second microelectrode (120) using the dielectrophoresis, molecules may be moved to a direction where the electrical field gradient is great, or to a direction where the electrical field gradient is small in response to the shape formed between the first microelectrode (110) and the second microelectrode (120).

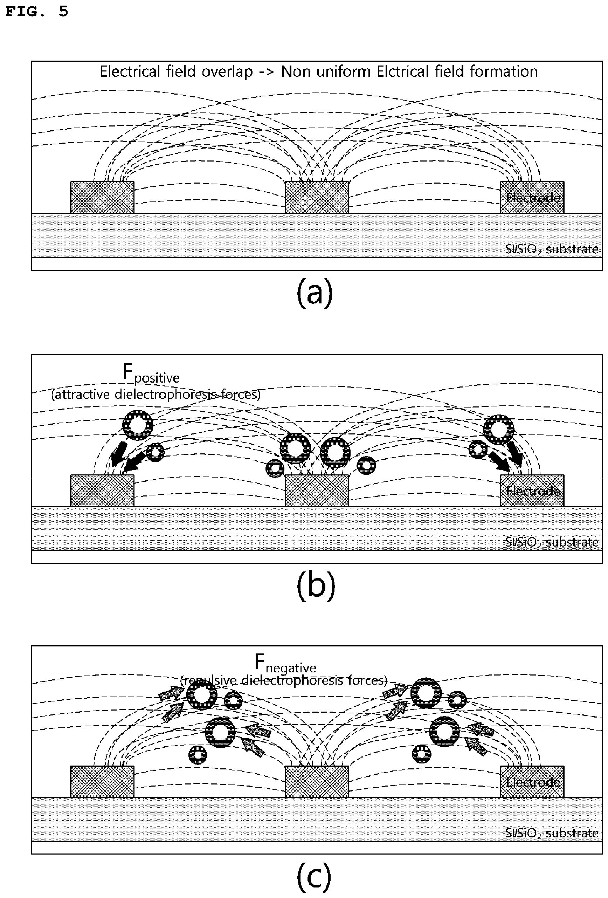

FIG. 5 illustrates examples explaining a particle movement state according to positive and negative electrophoresis, where a voltage is applied to between the first microelectrode (110) and the second microelectrode (120), a non-uniform electrical field may be formed between the first microelectrode (110) and the second microelectrode (120), as shown in FIG. 5a, to induce the di-electrophoresis forces.

FIG. 5b illustrates a phenomenon where molecules are moved to where the electrical field gradient is great (surface portion of electrode) by an induced positive (+) dielectrophoresis forces in response to the formed shape of electrical field, the phenomenon of which is called focusing of particles.

Conversely, FIG. 5c illustrates a phenomenon where molecules are moved to where the electrical field gradient is small (between electrodes) by an induced negative (-) dielectrophoresis forces in response to the formed shape of electrical field, the phenomenon of which is called trapping of particles.

As noted above, when a voltage is applied to the first microelectrode (110) and the second microelectrode (120) to allow the negative (-) dielectrophoresis forces to be generated in response to non-uniform electrical field shape between the first microelectrode (110) and the second microelectrode (120) and its gradient thereof, molecules may be moved and concentrated by the induced di-electrophoresis forces.

Particularly, the target biomaterials may be reacted by concentration thereof by moving the target biomaterials to where the electrical field gradient is small (between electrodes) using the negative (-) dielectrophoresis forces in response to the formed electrical field shape.

That is, quantitative analysis of target biomolecules may be realized by fixing receptors specifically binding to target biomolecules to between the first microelectrode (110) and the second microelectrode (120) in order to detect the moving biomaterials, and by ascertaining impedance change when the target biomolecules react to the receptors.

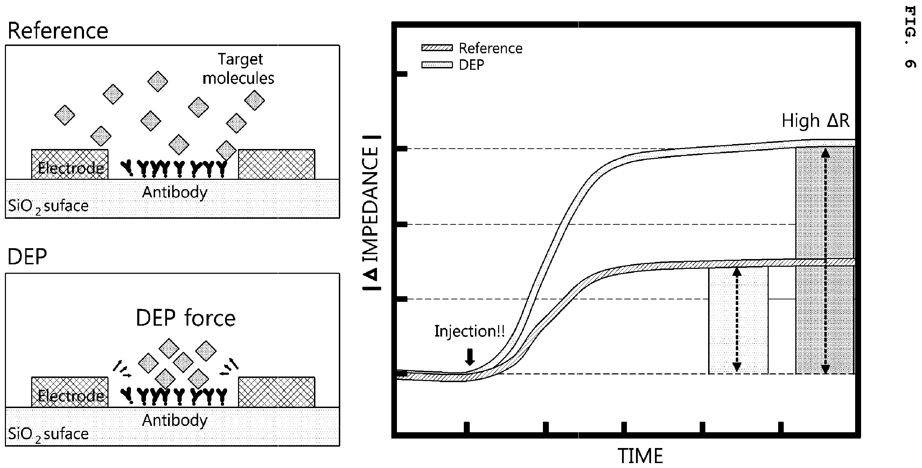

FIG. 6 illustrates a phenomenon where impedance change of biosensor is increased by inducing biomolecule concentration effect (negative $K(\omega)$ value) in which the target biomaterials are collected to where receptors are formed using the di-electrophoresis forces.

The impedance signal is increased due to collection of target biomolecules within a sample compared to a case where the dielectrophoresis forces are not acted, which leads to sensitivity enhancement of biosensor (100). Furthermore, a greater effect can be demonstrated under plasma or serum environment because an effect of effectively generating inhibition of non-specific binding formation is generated within a sample.

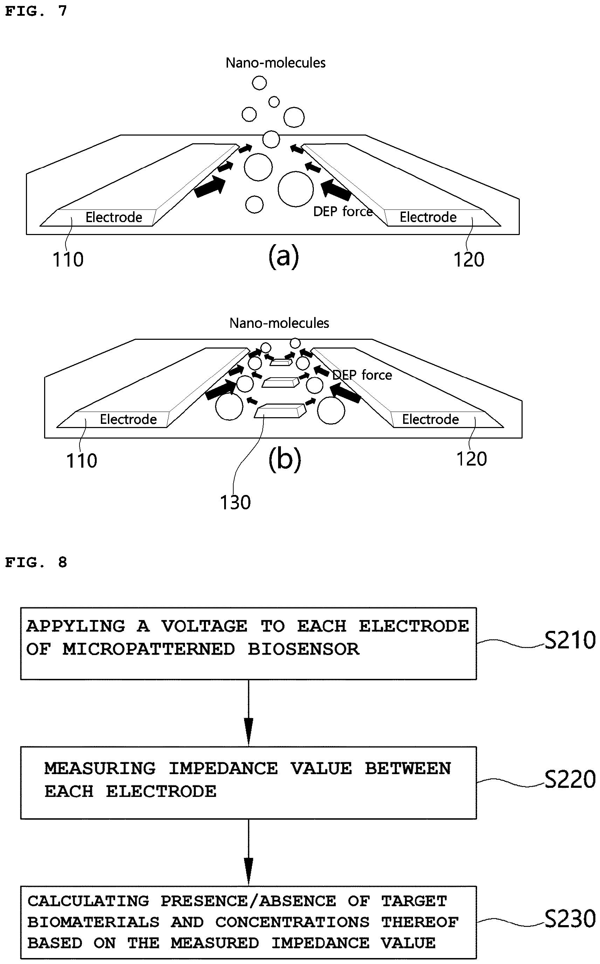

FIG. 7a illustrates an example of micropattern-less biosensor between the first microelectrode (110) and the second microelectrode (120) and FIG. 7b illustrates an example of micropattern (130)-existent biosensor between the first microelectrode (110) and the second microelectrode (120).

As shown in FIG. 7b, the non-uniform AC electric field is formed not only on the surface of biosensor (100) but also on the surface of micropattern (130) to allow generating a greater dielectrophoresis forces.

The dielectrophoresis forces may be increased by adjusting a distance between electrodes inside the biosensor and the size of non-uniform electric field formed through changes in intensity of applied voltage.

Particularly, the lowering of voltage necessary for concentration of biomolecules can prevent the damage to biomolecules caused by voltage applied for di-electrophoresis effect, and integration with battery or communication elements can be further eased when the biosensor is commercialized as a disease diagnosis health care sensor in the future as well.

Now, referring to FIG. 8, a method for detecting a biomaterial using a biosensor according to an exemplary embodiment of the present invention will be explained.

First of all, a voltage is applied to each microelectrode of biosensor in order to generate the dielectrophoresis forces (S210). Here, the biosensor means the biosensor (100) formed with micropatterns between the first microelectrode and the second microelectrode as shown in each exemplary embodiment thus explained.

The voltage applied in the step S210 is an AC voltage for generating the di-electrophoresis forces, and may be about 0.3V, and a frequency may be about 50MHz. However, the present invention is not limited thereto. For example, the AC voltage for generating the d dielectrophoresis forces may be greater than 0.25V but less than 0.35V.

When an AC voltage is applied at the step S210, the target biomaterials may be moved to where the electric field gradient is small (between each electrode) in response to the formed shape of electric field by the dielectrophoresis forces, and the target biomaterials may be concentrated between the electrodes.

Now, an impedance may be measured between each electrode (S220).

Then, presence or absence of target biomaterials or concentration thereof may be calculated (S230) based on the impedance values measured at step S220.

The method of calculating the presence or absence of target biomaterials or concentration thereof at the step S230 may be made in various ways.

The target biomaterials to be detected by the present invention may include various types, and particularly, may be a biomaterial with a weight of about 4.5kDa. However, the present invention is not limited thereto. For example, a weight of target biomaterial to be detected by the present invention may be greater than 4.0kDa but less than 5.0kDa. Here, an example of biomaterial with a weight of about 4.5kDa may include an amyloid beta protein.

Meantime, a position condition for collecting amyloid beta between each electrode may be determined by an applied voltage.

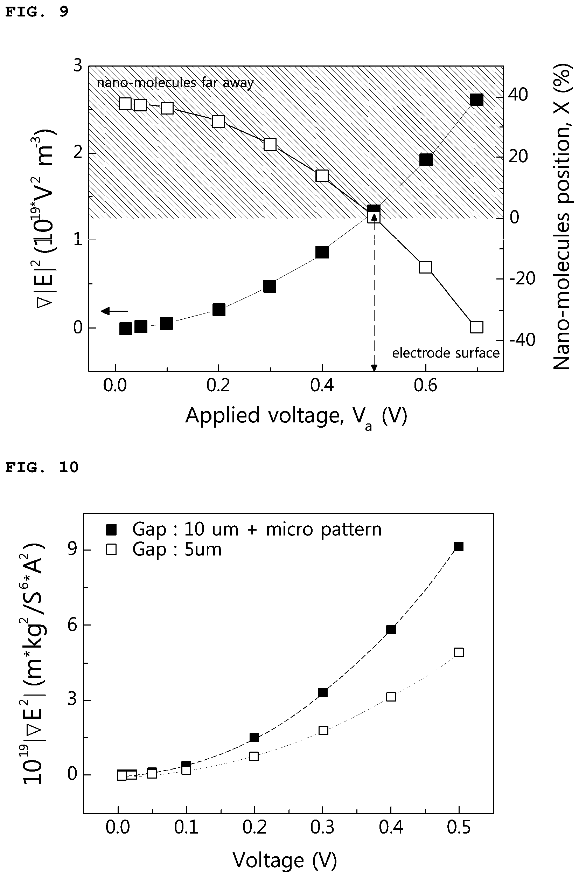

FIG. 9 illustrates an example of an IDE sensor where no micropattern exists between each electrode, where it is expected that a biomaterial with a weight of about 4.5 kDa having a size similar to that of amyloid beta is to be positioned at a square center between electrodes when an AC signal having 50 MHz and 0.5V is applied.

It should be apparent that the biosensor according to the present invention may be applied to detect a protein heavier than the said amyloid beta protein or a protein lighter than the said amyloid beta protein, which can be made possible by changing and/or adjusting a voltage condition in response to weight of protein to be detected.

FIG. 10 illustrates an intensity of non-uniform AC electric field formed by a voltage applied in response to presence or absence of microelectrodes,
As expected from FIG. 9, the target biomaterial (e.g., amyloid beta) may be positioned at a square center between electrodes when a voltage applied to each electrode is at 0.5V in the micropattern-less biosensor, and at this time, the intensity of electric field may be shown.

Meantime, it can be known that an electric field greater than that of the micropattern-less biosensor having an electrode width of 5$\mu$m under the same applied voltage is formed, even if a distance between electrodes comes to be increased to 10$\mu$m in the micropattern-existent biosensor.

Now, an experimental example related to the biosensor (100) according to the present invention will be explained.

The present experiment is to ascertain a possibility of enhancing the performance of the biosensor by implementing a qualitative analysis in order to detect amyloid beta existent within a serum.

Toward this end, an optimal voltage condition has been established in order to concentrate the amyloid beta within the biosensor (100) disposed with a micropattern.

A biosensor has been manufactured using a batch process-enabled MEMS (Micro Electro Mechanical System) process, and a sensor surface activation for fixation of antibody has been proceeded. Sensitivity of biosensor has been compared through quantitative analysis of amyloid beta within 1x PBS buffer using a biosensor having various micropattern structures, and quantitative analysis of amyloid beta existent within serum has been made using optimized biosensor.

**(1) Manufacturing of micropatterned IDE microelectrode sensor**

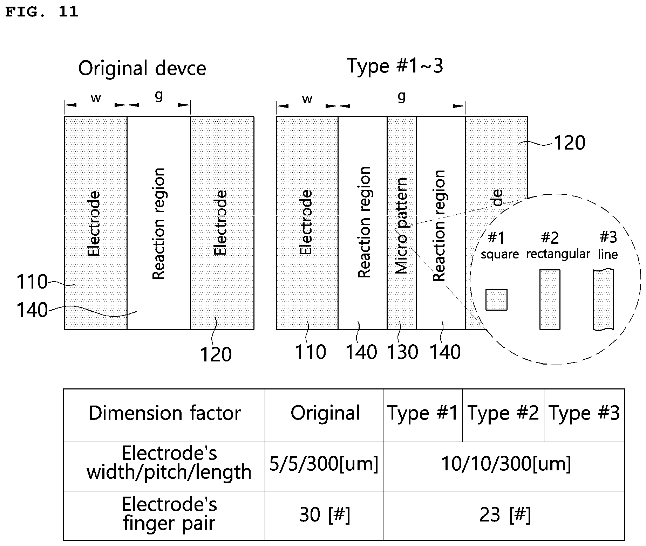

As shown in an example of FIG. 11, micropatterns of three different structures have been designed in order to ascertain the performance of micropatterned biosensor.

FIG. 11 illustrates a schematic structural diagram of a micropattern-less biosensor (indicated as an Original Device) and micropatterned biosensors (Type #1~3), thickness of electrode, width, length and the number of electrodes.

The micropattern (130) has been respectively manufactured in a square structure with each side of 2.5μm, a rectangular structure with one side of 2.5μm and the other side of 7.5μm, and a linear structure with a width of 2.5μm. The Type #1 shows a square-shaped micropatterned biosensor, the Type #2 shows a rectangular micropatterned biosensor and the Type #3 shows a linear micropatterned biosensor.

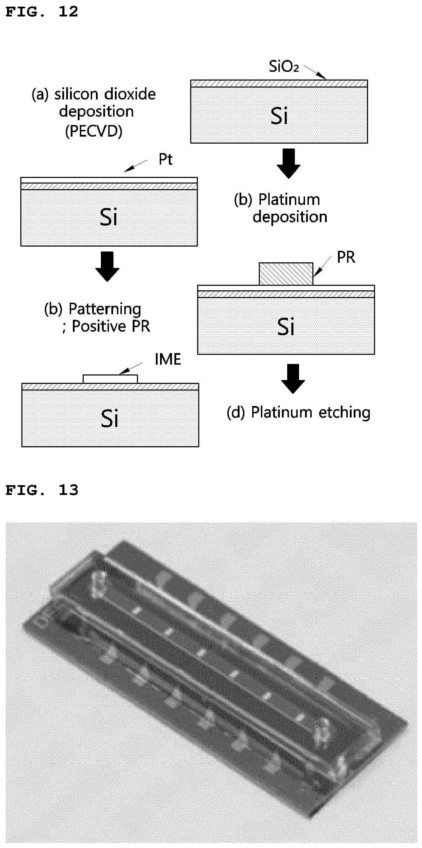

As shown in FIG. 12, the biosensor has been manufactured using the MEM process, and an SiO2 film-deposited silicon (Si) wafer has been deposited with Pt (Platinum) film with a 150nm thickness using sputtering method (a)(b). Furthermore, patterning has been made (c) using a mask manufactured through photolithography process after Pt film deposition, and the pattern has been completed using a dry etching method (d).

FIG. 13 is a real physical example of a biosensor manufactured according to the present invention, where a unit chip is disposed with six (6) biosensors, which is to minimize errors that may be generated during analysis of same sample.

As a result, integration becomes easy that forms a plurality of IDE sensors on one chip through a mask manufacturing in light of characteristics of MEMS process, whereby a multiple analysis is made possible that is capable of analyzing various proteins from one chip.

**(2) sensor surface activation through antibody fixation**

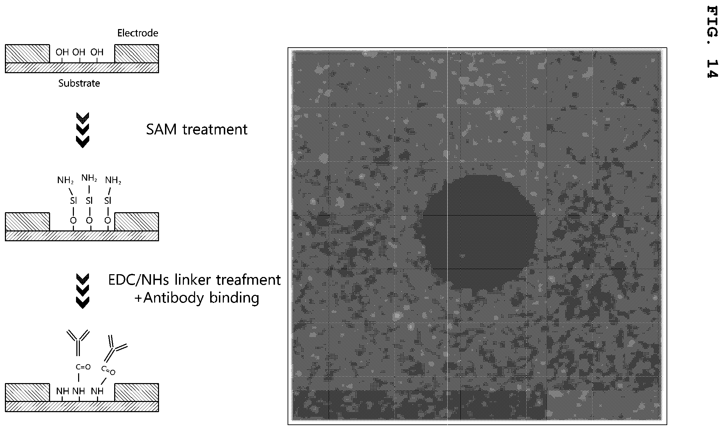

FIG. 14 is a schematic view on surface treatment method through antibody fixation, and shows a surface of biosensor fixed by an antibody after surface treatment, where APMEMS of Vapour Phase has been used in order to form an SAM (Self-Assembled Monolayer) layer on an SiO2 surface activated by '-OH' linker between each microelectrode, and 'EDC/NHs' has been used as linker in order to fix the antibody after the formation of SAM layer.

It has been confirmed/ascertained that antibody had been fixed through brightness difference between a portion within a circle and a portion outside of the circle. Furthermore, it has been also ascertained that the antibody had been uniformly fixed across the entire surface-treated region through the brightness at a portion outside of the circle being uniform across the surface.

**(3) Ascertainment of possibility of biomolecule concentration having micropattern**

In light of the fact that, in order to evaluate the performance of biosensor disposed with a micropattern, the first thing is that a surface activation condition must be applied and an effect of the dielectrophoresis forces must be confirmed, an optimal condition that may shows the dielectrophoresis effect must be established, and therefore, experiments of qualitative analysis have been performed using these prerequisites.

Toward this end, characteristics of amyloid beta with a weight of about 4.5 kDa has been ascertained and a voltage condition for generating the di-electrophoresis effect has been confirmed by way of simulation.

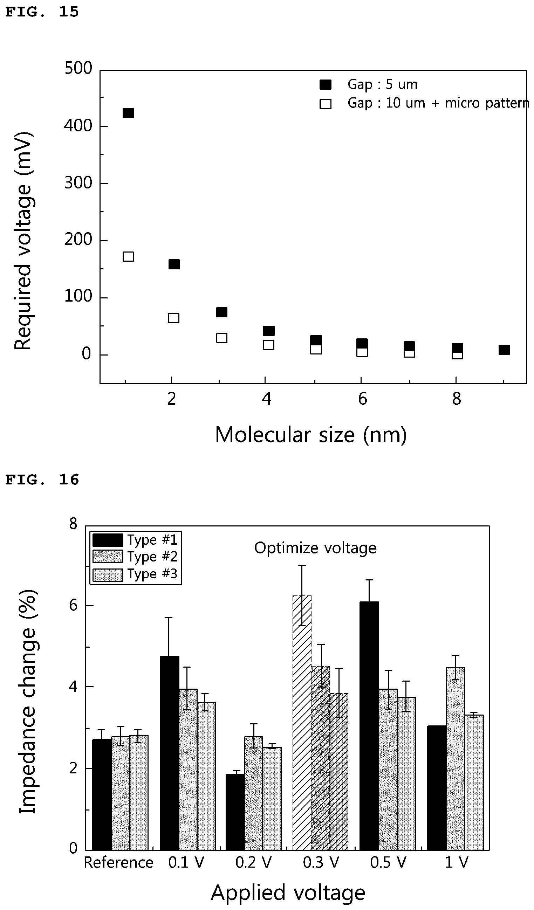

FIG. 15 illustrates an example showing a dielectrophoresis voltage condition for collecting the biomolecules between each microelectrode based on sizes thereof. As shown in FIG. 15, it is estimated that the dielectrophoresis forces may act in the vicinity of about 0.2V in order to concentrate the amyloid beta between the electrodes in case of micropattern-existent biosensor.

Therefore, the presence and/or absence of amyloid beta concentration have been confirmed using five (5) ambient voltages of 0.1, 0.2, 0.3, 0.5 and 1.0V.

The confirmation experiment has confirmed the effect by allowing the antibody to be fixed on the biosensor using the same protocol, and by applying dielectrophoresis voltage and frequency conditions corresponding to each condition while reacting amyloid beta protein of 10 pg/mL, where the same frequency of 50MHz was applied.

As shown in FIG. 16, it was confirmed that the impedance change was greatly demonstrated when a voltage of 0.3V was applied, and this condition has been utilized as an applied signal condition during the qualitative analysis.

It was confirmed that the size of the optimized dielectrophoresis voltage was an about 50% reduced value over the 0.5V used in the IDE sensor (micropattern-less biosensor) having a distance of 5μm between electrodes.

## (4) Sensitivity evaluation for each sensor structure through biomolecule quantitative analysis

A quantitative analysis has been made on amyloid beta protein using the established dielectrophoresis voltage condition, and preparation has been made by melting, in 1xPBS, the amyloid beta sample increased in concentration by 10 times from 100 fg/mL to 100 pg/mL.

The analysis process has been made using three (3) steps including: a signal stabilization inside the buffer; reaction and application of signal for dielectrophoresis; and washing and signal stabilization.

As illustrated in FIG. 7, when amyloid beta proteins are collected between each electrode using the dielectrophoresis effect, an about 3% ~ 5% impedance change has been noticed in response to amyloid beta protein concentration in the micropattern-less IDE sensor.

Meantime, an increase of 0.5% or more in the entire impedance change value has been noticed regardless of micropattern structure in case of micropatterned biosensor. That is, there has been an impedance change of 3.5% ~ 5.5% depending on the amyloid beta protein concentration.

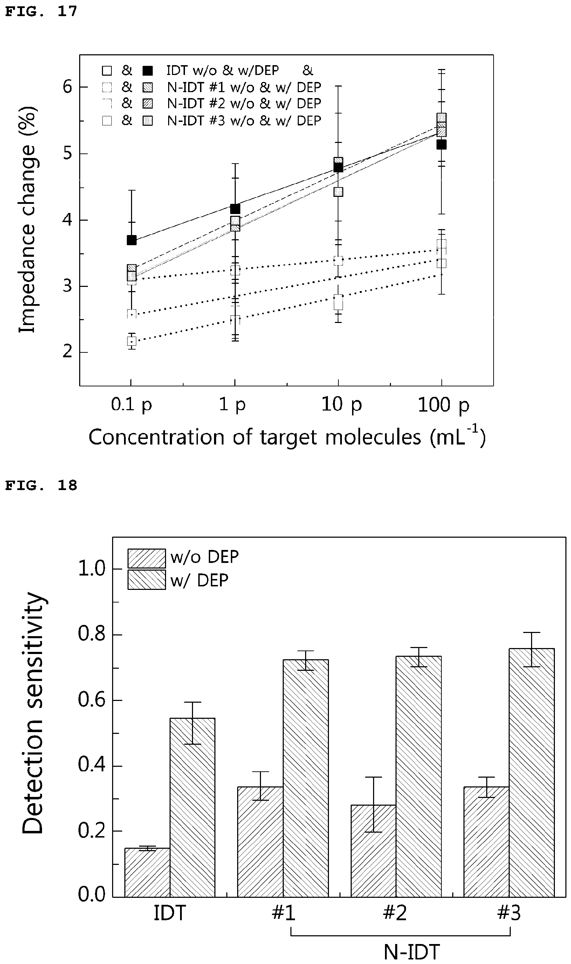

FIG. 18 illustrates the sensitivity (qualitative analysis gradient, dZ/Conc.) of biosensor in response to dielectrophoresis effect, where sensitivity has been compared based on use or non-use of dielectrophoresis phenomenon for each type of biosensor.

The sensitivity has been increased from $0.146 \pm 0.005$ to $0.545 \pm 0.049$ by the dielectrophoresis effect in the micropattern-less biosensor (IDT).

On the other hand, in the micropattern-formed biosensor and depending on micropattern structure, it has been confirmed that the sensitivity had been increased from $0.337 \pm 0.043$ to $0.724 \pm 0.027$ in the Type #1 (square pattern), the sensitivity had been increased from $0.281 \pm 0.083$ to $0.731 \pm 0.033$ in the Type #2 (rectangular pattern), and the sensitivity had been increased from $0.337 \pm 0.031$ to $0.757 \pm 0.051$ in the Type #3 (linear pattern).

It can be noted through these experiments that the micropattern-existent IDE sensor has demonstrated a higher sensitivity than that of micropattern-less IDE sensor due to the dielectrophoresis effect.

Each result from the present experiments has been measured under an environment inside the PBS buffer, and it has been confirmed that the structure of the most optimized biosensor is Type #3 formed with the line(ar) micropattern, with sensitivity thereof being of $0.757 \pm 0.051$, detection limit being of 100 fg/mL, detection section (dynamic Range) being of 100 pg/mL at 100 fg/mL.

## (5) Quantitative evaluation of biomolecule under standard plasma environment

Amyloid beta quantitative analysis has been performed within a plasma using an amyloid beta quantitative analysis sensor based on the optimized dielectrophoresis effect, and the type of biosensor used herein was the Type #3 (sensor using linear micropattern).

In order to perform amyloid beta quantitative analysis within a plasma, a preparation has been made by melting, in plasma, the amyloid beta sample increased in concentration by 10 times from 100 fg/mL to 100 pg/mL.

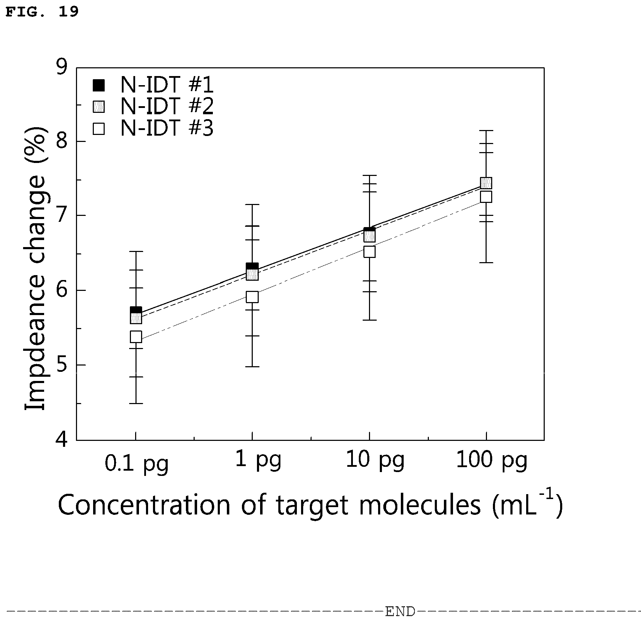

The same analysis method has been used as the amyloid beta analysis process within 1x PBS as explained above. As a result of the analysis, as shown in FIG. 19, when the amyloid beta quantitative analysis was performed within the plasma using the optimized biosensor, it has been confirmed that impedance change had been made due to about 5.5~7.5% of amyloid beta.

It has been confirmed through these experiments that, when amyloid beta within the plasma was analyzed, It has been confirmed that the sensitivity of optimized biosensor had been $0.628 \pm 0.032$, detection limit had been 100 fg/mL, and the detection section had been 100 pg/mL at 100 fg/mL.

The sensitivity of sensor during amyloid beta analysis within a plasma had been about 83% of the sensitivity confirmed while analyzed within the PBS, which is estimated as a decrease in sensitivity due to various biomaterials existent within the plasma, but the said sensitivity has shown a remarkably excellent performance over the conventional detection techniques, and therefore, may be usefully utilized for biomarker detection within an actual sample.

While the present invention has been described with reference to the particular illustrative embodiments, it is not to be restricted by the embodiments but only by the appended claims. It is to be appreciated that those skilled in the art can change or modify the embodiments without departing from the scope and spirit of the present invention.

Therefore, it should be understood that the abovementioned exemplary embodiments are simply exemplary but not restrictive. For example, each element described in a single form may be a dispersed manner, and likewise, each element described in a dispersed manner may be implemented in a combined manner.

It should be interpreted that the scope of the present invention will be described from the following claims rather than the foregoing detailed description, and all other types of changes or modifications derived from the meaning of claims, scopes and equivalent shapes are included in the present invention.

[Description of Reference Numerals]

100:    biosensor

110:    first microelectrode

120:    second microelectrode

130:    micropattern

## Claims

1. A dielectrophoresis-used microelectrode biosensor comprising:

   a first microelectrode with a plurality of first protruding electrodes arranged in a comb shape on a substrate;
   a second microelectrode arranged to intersect with each first protruding electrode formed on the first microelectrode and arranged with a plurality of second protruding electrodes arranged in a comb shape;
   a conductive-materialed micropattern formed in a space between the first microelectrode and the second microelectrode; and
   a plurality of receptors fixed in a space between the first microelectrode and the second microelectrode to specifically react with a target biomaterial.

2. The dielectrophoresis-used microelectrode biosensor according to claim 1, wherein the first microelectrode and the second microelectrode are applied with an AC (Alternating Current) for generating a dielectrophoresis force.

3. The dielectrophoresis-used microelectrode biosensor according to claim 1, wherein the micropattern is formed in any one shape of square, rectangular and line(ar) shapes.

4. The dielectrophoresis-used microelectrode biosensor according to claim 3, wherein length of each side of the square is greater than $2.0\mu m$ but less than $3.0\mu m$.

5. The d dielectrophoresis-used microelectrode biosensor according to claim 3, wherein length of one side of the square is greater than $2.0\mu m$ but less than $3.0\mu m$ while length of the other side of the square is greater than $7.0\mu m$ but less than $8.0\mu m$.

6. The dielectrophoresis-used microelectrode biosensor according to claim 3, wherein width of line(ar) micropattern is greater than $2.0\mu m$ but less than $3.0\mu m$.

7. The dielectrophoresis-used microelectrode biosensor according to claim 1, wherein intensity of AC generating the dielectrophoresis is greater than 0.25V but less than 0.35V, and frequency is 50MHz.

8. The dielectrophoresis-used microelectrode biosensor according to claim 1, wherein the target biomaterial includes amyloid beta protein, and the receptor includes amyloid beta antibody.

9. A method for detecting bio material using a biosensor, comprising: applying a voltage to the first microelectrode and the second microelectrode using the dielectrophoresis according to any one preceding claim of 1 to 8; measuring an impedance between the first microelectrode and the second microelectrode; and calculating one or more of presence or absence of target biomaterial and concentration thereof based on the measured impedance.

10. The method according to claim 9, wherein the voltage applied between the first microelectrode and the second microelectrode is an AC for generating a dielectrophoresis force, and the magnitude of the voltage is greater than 0.25V but less than 0.35V, and frequency is 50MHz.

11. The method according to claim 9, wherein weight of the target bio material is greater than 4.0kDa but less than 5.0kDa.

12. The method according to claim 9, wherein the target biomaterial includes amyloid beta protein.

FIG. 1

FIG. 2

100

110    120

(a)

110

140

130

140

120

(b)

FIG. 3

110 — 120

131

(a) SQUARE PATTERN

110 — 120

132

(b) RECTANGULAR PATTERN

110 — 120

133

(c) LINE (AR) PATTERN

FIG. 4

$F_{positive}$

Eloctrode

Nano-particle

Eloctrode

(a)

Nano-particle

Eloctrode

$F_{negative}$

Eloctrode

(b)

FIG. 5

(a)

(b)

(c)

FIG. 6

FIG. 7

Nano-molecules

(a)

Nano-molecules

(b)

FIG. 8

| |
|---|
| APPYLING A VOLTAGE TO EACH ELECTRODE OF MICROPATTERNED BIOSENSOR |

~S210

| |
|---|
| MEASURING IMPEDANCE VALUE BETWEEN EACH ELECTRODE |

~S220

| |
|---|
| CALCULATING PRESENCE/ABSENCE OF TARGET BIOMATERIALS AND CONCENTRATIONS THEREOF BASED ON THE MEASURED IMPEDANCE VALUE |

~S230

FIG. 9

FIG. 10

FIG. 11

Original devce

Type #1~3

| Dimension factor | Original | Type #1 | Type #2 | Type #3 |
|---|---|---|---|---|
| Electrode's width/pitch/length | 5/5/300[um] | 10/10/300[um] | | |
| Electrode's finger pair | 30 [#] | 23 [#] | | |

**FIG. 12**

(a) silicon dioxide
deposition
(PECVD)

SiO₂

Si

(b) Platinum
deposition

Pt

Si

(b) Patterning
; Positive PR

PR

Si

IME

Si

(d) Platinum etching

**FIG. 13**

**FIG. 14**

FIG. 15

FIG. 16

**FIG. 17**

**FIG. 18**

FIG. 19

## INTERNATIONAL SEARCH REPORT

| International application No. |
|---|
| PCT/KR2019/008461 |

**A. CLASSIFICATION OF SUBJECT MATTER**

*G01N 27/327(2006.01)i, G01N 27/447(2006.01)i, G01N 33/543(2006.01)i*

According to International Patent Classification (IPC) or to both national classification and IPC

**B. FIELDS SEARCHED**

Minimum documentation searched (classification system followed by classification symbols)
G01N 27/327; G01N 27/02; G01N 27/22; G01N 27/30; G01N 27/447; G01N 33/483; G01N 33/50; G01N 33/543

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal) & Keywords: micro electrode, micro pattern layer, impedance, acceptor(contain unit), target biomaterial

**C. DOCUMENTS CONSIDERED TO BE RELEVANT**

| Category* | Citation of document, with indication, where appropriate, of the relevant passages | Relevant to claim No. |
|---|---|---|
| Y | KR 10-1727107 B1 (KOREA INSTITUTE OF SCIENCE AND TECHNOLOGY) 17 April 2017 See paragraphs [0038]-[0046], claims 1, 5, figure 1. | 1-12 |
| Y | KR 10-2015-0079147 A (SEOUL NATIONAL UNIVERSITY R&DB FOUNDATION) 08 July 2015 See paragraphs [0039]-[0040], claim 1 and figure 1. | 1-12 |
| Y | KR 10-2016-0034434 A (KOREA INSTITUTE OF SCIENCE AND TECHNOLOGY et al.) 30 March 2016 See paragraph [0048], claim 1 and figures 1, 4. | 1-12 |
| A | KR 10-2009-0033166 A (DHIRANI, Al-amin et al.) 01 April 2009 See claim 1 and figures 1-2. | 1-12 |
| A | US 2014-0014536 A1 (THE REGENTS OF THE UNIVERSITY OF CALIFORNIA) 16 January 2014 See paragraphs [0050]-[0051] and figure 6B. | 1-12 |

☐ Further documents are listed in the continuation of Box C. ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 06 NOVEMBER 2019 (06.11.2019) | **06 NOVEMBER 2019 (06.11.2019)** |

| Name and mailing address of the ISA/KR | Authorized officer |
|---|---|
| Korean Intellectual Property Office Government Complex Daejeon Building 4, 189, Cheongsa-ro, Seo-gu, Daejeon, 35208, Republic of Korea Facsimile No. +82-42-481-8578 | Telephone No. |

Form PCT/ISA/210 (second sheet) (January 2015)

**INTERNATIONAL SEARCH REPORT**
Information on patent family members

International application No.

**PCT/KR2019/008461**

| Patent document cited in search report | Publication date | Patent family member | Publication date |
|---|---|---|---|
| KR 10-1727107 B1 | 17/04/2017 | US 2019-0041357 A1<br>WO 2017-142166 A1 | 07/02/2019<br>24/08/2017 |
| KR 10-2015-0079147 A | 08/07/2015 | KR 10-1551876 B1 | 09/09/2015 |
| KR 10-2016-0034434 A | 30/03/2016 | KR 10-1648383 B1<br>WO 2016-043402 A1 | 24/08/2016<br>24/03/2016 |
| KR 10-2009-0033166 A | 01/04/2009 | CA 2643354 A1<br>CA 2643354 C<br>CN 101443656 A<br>CN 101443656 B<br>EP 2005148 A1<br>EP 2005148 B1<br>HK 1129928 A1<br>JP 2009-529683 A<br>US 2009-0273354 A1<br>US 8246910 B2<br>WO 2007-104163 A1 | 20/09/2007<br>26/04/2016<br>27/05/2009<br>14/08/2013<br>24/12/2008<br>07/03/2018<br>04/04/2014<br>20/08/2009<br>05/11/2009<br>21/08/2012<br>20/09/2007 |
| US 2014-0014536 A1 | 16/01/2014 | CA 2646987 A1<br>CA 2646987 C<br>EP 1996734 A2<br>EP 1996734 B1<br>ES 2691647 T3<br>US 2009-0092965 A1<br>US 8513001 B2<br>US 9316608 B2<br>WO 2007-104058 A2<br>WO 2007-104058 A3 | 13/09/2007<br>30/09/2014<br>03/12/2008<br>01/08/2018<br>28/11/2018<br>09/04/2009<br>20/08/2013<br>19/04/2016<br>13/09/2007<br>06/11/2008 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 101727107 A

- KR 1727107